# EUROPEAN PATENT SPECIFICATION

(11) **EP 3 047 801 B1**
(45) Date of publication and mention of the grant of the patent: **11.04.2018**
(21) Application number: 15173958.8
(22) Date of filing: 26.06.2015
(51) Int. Cl.: A61B 8/00

(54) **ULTRASOUND IMAGING APPARATUS AND CONTROLLING METHOD OF ULTRASOUND IMAGING APPARATUS**
ULTRASCHALLBILDGEBUNGSVORRICHTUNG UND STEUERUNGSVERFAHREN EINER ULTRASCHALLBILDGEBUNGSVORRICHTUNG
APPAREIL D'IMAGERIE À ULTRASONS ET PROCÉDÉ DE COMMANDE D'UN APPAREIL D'IMAGERIE À ULTRASONS

(30) Priority: 20.01.2015 KR 20150009022
(43) Date of publication of application: 27.07.2016
(73) Proprietor: Samsung Medison Co., Ltd., Gangwon-do 250-870 (KR)
(72) Inventor: Lee, Sang-Mok, Seoul (KR); Kim, Nam-Woong, Seoul (KR); Lee, Hong-Gyo, Seoul (KR)
(74) Representative: Frey, Sven Holger

(56) References cited:
- JP-A- 2012 223 353
- US-A1- 2010 292 577
- US-A1- 2013 165 790

## Description

### BACKGROUND

### 1. Field

Apparatuses and methods consistent with exemplary embodiments relate to a probe assembly, an ultrasound imaging apparatus configured to generate an ultrasound image, and a controlling method the ultrasound imaging apparatus.

### 2. Description of Related Art.

An ultrasound imaging apparatus transmits an ultrasonic signal from the surface of an object to the object, detects an ultrasonic signal reflected from the object, i.e., echo ultrasonic waves, generates an internal image of the object such as a tomogram of a soft tissue or bloodstream, and provides information related to the target part.

An ultrasound imaging apparatus is compact, inexpensive, noninvasive, and nondestructive in comparison with another type of diagnostic imaging apparatus, e.g., X-ray device, Computerized Tomography (CT) scanner, Magnetic Resonance Image (MRI), diagnostic nuclear medical apparatus. Therefore, the ultrasound imaging apparatus is widely used in medical examination at maternity, cardiology, abdomen, and urology clinics.

An ultrasound imaging apparatus includes a body in which main components of the ultrasound imaging apparatus are included, a probe transmitting an ultrasonic signal to an object and receiving echo ultrasonic signal reflected from the object to acquire an ultrasound image and a probe assembly including a probe connector connected to the body.

Document US2010292577 A1 describes an ultrasound image diagnosis apparatus executing lighting-on and lighting-off of a lighting device by detecting an identification (ID) data of a probe or an operation on an operation panel.

Document JP2012223353 A describes an ultrasonic diagnosis apparatus that prevents a probe that is in use to be removed from the apparatus body.

Document US2013165790 A1 describes an ultrasound diagnostic apparatus that facilitates fastening between a main body and a probe.

### SUMMARY

Therefore, it is an aspect of the present disclosure to provide a probe assembly provided with an identification information transmitter configured to transmit identification information to a body.

It is another aspect of the present disclosure to provide an ultrasound imaging apparatus provided with an identification information receiver configured to receive identification information from a probe assembly, and a control method of the ultrasound imaging apparatus.

Additional aspects of the present disclosure will be set forth in part in the description which follows and, in part, will be obvious from the description, or may be learned by practice of the invention.

In accordance with one aspect of the present disclosure, an ultrasound imaging apparatus includes a placing unit in which at least one probe connector is placed, an identification information receiver receiving probe assembly identification information from the placed probe connector, an input device receiving a selection command of at least one probe connector, and a locking unit locking the at least one probe connector with the ultrasound imaging apparatus based on the selection command.

The identification information receiver may include a second contact point receiving the probe assembly identification information by making contact with a first contact point placed in the probe connector.

The second contact point may make contact with the first contact point by manual operation of a user.

The locking unit may lock the probe connector into the ultrasound imaging apparatus so that the first contact point makes contact with the second contact point.

The identification information receiver may include a second wireless communication module receiving the probe assembly identification information by being connected to a first wireless communication module of the probe connector.

The locking unit may lock the probe connector into the ultrasound imaging apparatus so that the first wireless communication module makes contact with the second wireless communication module.

The ultrasound imaging apparatus may further include a display device displaying the probe assembly identification information.

The locking unit may perform a first locking connecting the identification information transmitter of the probe connector to the identification information receiver of the ultrasound imaging apparatus, and a second locking connecting a probe signal transceiver of the probe connector to a body signal transceiver of the ultrasound imaging apparatus.

The locking unit may release the first locking between a probe connector, which is not selected from the plurality of probe connectors, and the ultrasound imaging apparatus.

When the input device receives whether to select a first probe assembly, and then the first probe assembly is selected, the locking unit may perform a second locking between the first probe assembly and the ultrasound imaging apparatus, and when the first probe assembly is not selected, the locking unit may perform the first locking between a second probe assembly and the ultrasound imaging apparatus.

When the first probe assembly is not selected, the locking unit may release the first locking between the first probe assembly and the ultrasound imaging apparatus.

The ultrasound imaging apparatus may further include a controller generating a control signal to control the locking unit, wherein the locking unit may include a motor or an actuator, both of which perform the first locking or the second locking based on the control signal.

The placing unit may be realized by an internal screw type, and the locking unit may perform the first locking or the second locking by rotary coupling a housing of the probe assembly realized by an external screw type to the placing unit realized by an internal screw type.

The placing unit may be realized by a coupling groove forming member, in which coupling grooves are formed so that a locking shaft of the probe assembly is placed.

Locking grooves, in which a locking pin of the locking shaft is inserted, may be formed in the coupling groove forming member, and the locking unit may include a coupling member provided at a position formed by an extension of the coupling groove and a motor rotating the locking shaft placed in the coupling member, wherein the motor may perform locking by inserting the locking pin into the locking groove by rotating the locking shaft.

The locking unit may include an electromagnet and a driving device wherein the driving device may perform locking by moving the electromagnet.

In accordance with another aspect of the present disclosure, a probe connector includes a storage unit storing probe assembly identification information, an identification information transmitter transmitting the probe assembly identification information to an ultrasound imaging apparatus when placed placed in the ultrasound imaging apparatus, and a probe signal transceiver connected to a body signal transceiver of the ultrasound imaging apparatus.

The storage unit may include an electronic tag.

The identification information transmitter may include a first contact point transmitting the probe assembly identification information by making contact with a second contact point placed placed inin the ultrasound imaging apparatus.

The identification information transmitter may include a first wireless communication module transmitting the probe assembly identification information by being connected to a second wireless communication module of the ultrasound imaging apparatus.

The probe signal transceiver may include a conductive connection pin.

In accordance with another aspect of the present disclosure, a control method of an ultrasound imaging apparatus includes an operation in which an identification information receiver receives probe assembly identification information from at least one probe connector, which is placed in a placing unit, receiving a selection command of at least one probe connector, and an operation in which a locking unit locks the at least one probe connector into an ultrasound imaging apparatus based on the selection command.

### BRIEF DESCRIPTION OF THE DRAWINGS

The above and/or other aspects will become more apparent by describing certain exemplary embodiments with reference to the accompanying drawings, in which:
FIG. 1 is a perspective view illustrating an external appearance of an ultrasound imaging apparatus in accordance with one embodiment of the present disclosure;
FIG. 2 is a view illustrating a configuration of a probe assembly;
FIGS. 3A to 3C are views illustrating various examples of a probe;
FIG.4 is a block diagram illustrating a probe assembly in accordance with one embodiment of the present disclosure;
FIG. 5 is a block diagram illustrating a body in accordance with one embodiment of the present disclosure;
FIGS. 6A to 7 are views illustrating an identification information transmitter and an identification information receiver in accordance with one embodiment of the present disclosure;
FIG. 8 is an external perspective view from front of a probe connector and a probe connection device;
FIG. 9 is an external perspective view from back of a probe connector;
FIG. 10 is an external perspective view from side of a probe connector;
FIGS. 11A to 11C are side views of a locking shaft and a body illustrating a locking method of a probe connector and a probe connection device in accordance with one embodiment of the present disclosure;
FIGS. 12 and 13 are external views of a probe connector and a probe connection device illustrating a locking method of a probe connector and a probe connection device in accordance with another embodiment of the present disclosure;
FIG. 14A is an exemplary view of a probe assembly identification information screen displayed by a display device;
FIG. 14B is an exemplary view illustrating a probe identification information screen displayed by a display device when placed plurality of probe connectors performs a first locking with each probe connection device in sequence;
FIG. 15 is a control block diagram of an ultrasound imaging apparatus in one embodiment of the present disclosure; and
FIG. 16 is a control block diagram of an ultrasound imaging apparatus in another embodiment of the present disclosure.

### DETAILED DESCRIPTION

Reference will now be made in detail to exemplary embodiments with reference to the accompanying drawings, wherein like reference numerals refer to like elements throughout. In this regard, the exemplary embodiments may have different forms and should not be construed as being limited to the descriptions set forth herein. Accordingly, the exemplary embodiments are merely described below, by referring to the figures, to explain aspects of the present description.

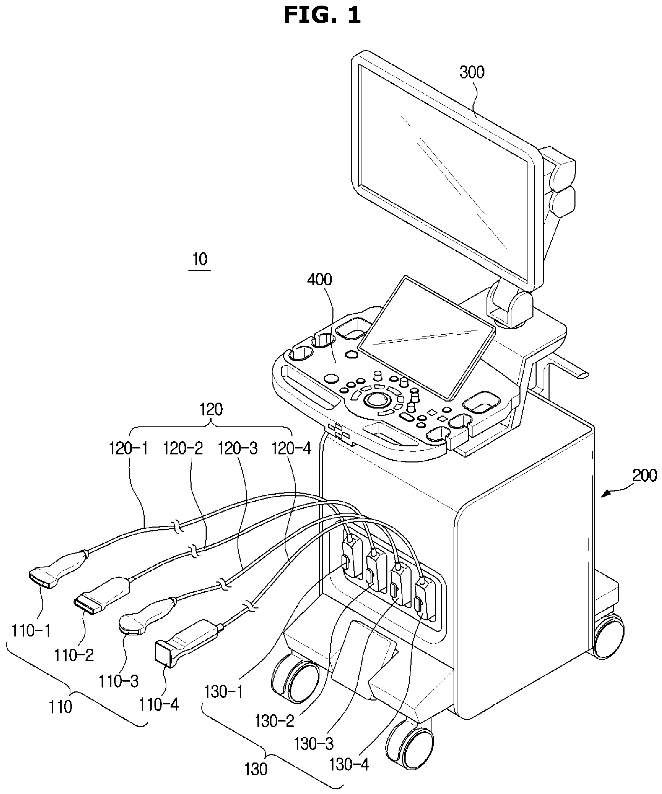

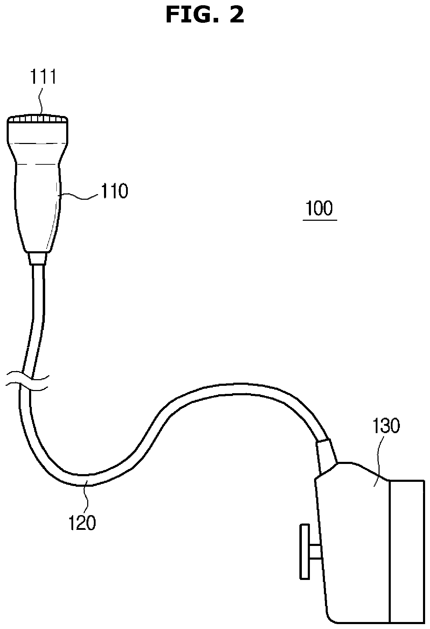

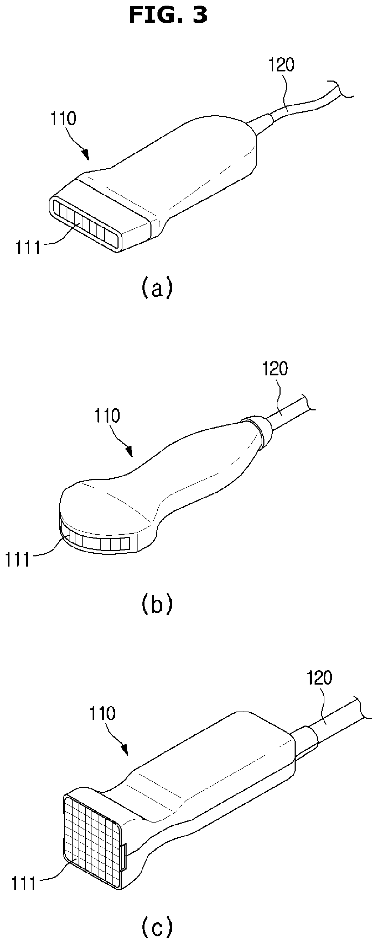

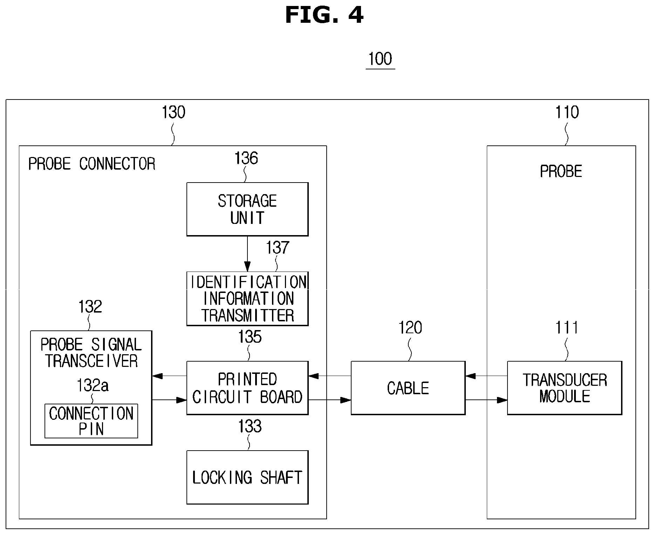

FIG. 1 is a perspective view illustrating an external appearance of an ultrasound imaging apparatus in accordance with one embodiment of the present disclosure, FIG. 2 is a view illustrating a configuration of a probe assembly, FIGS. 3A to 3C are views illustrating various examples of a probe, and FIG.4 is a block diagram illustrating a probe assembly in accordance with one embodiment of the present disclosure.

Referring to FIGS. 1 and 2, an ultrasound imaging apparatus 10 includes a probe assembly 100, a body 200, a display device 300, an input device 400. The probe assembly 100 illustrated in FIG. 2 may be any probe assembly 100 among a first probe assembly 100-1 to a fourth probe assembly 100-4, as illustrated in FIG. 1.

The probe assembly 100 transmits ultrasonic waves to an object, receives echo ultrasonic waves reflected from the object, and converts the echo ultrasonic waves into an electrical signal (hereinafter it will be referred to as an ultrasonic signal).

The probe assembly 100 placed in the body 200 of the ultrasound imaging apparatus 10 includes a plurality of probe assemblies, hereinafter a plurality of probe assemblies placed in the body 200 of the ultrasound imaging apparatus 10 will be described as the first probe assembly to the fourth probe assembly 100-1, 100-2, 100-3, and 100-4.

The body 200 generates an ultrasound image based on an ultrasonic signal.

The first probe assembly 100-1 to the fourth probe assembly 100-4 may be placed in the body 200, and the body 200 may be a work station provided with the display device 300 and the input device 400.

Hereinafter a detail configuration and an operating method of any probe assembly 100 among the first probe assembly 100-1 to the fourth probe assembly 100-4 will be described.

The probe assembly 100 includes a probe 110 making contact with an object, a probe connector 130 transmitting /receiving a signal to/from the body 200, and a cable 120 connecting the probe 110 to the probe connector 130.

The probe 110 may allow an ultrasound image of the inside of an object to be acquired by transmitting/receiving ultrasonic waves to/from the object.

Particularly, the probe 110 includes a transducer module 111 configured to convert an electrical signal into vibration energy or vibrational energy into an electrical energy, wherein the transducer module 111 transmits ultrasonic waves to the object and receives echo ultrasonic waves reflected from the object by using a vibrator, such as piezoelectrics (not shown). Hereinafter an electrical signal generated by the probe assembly 110 is referred to as "an ultrasonic signal".

For example, when the number of vibrators is approximately 64∼ approximately 256, a connection component is needed as many as the number of vibrators when the probe assembly 100 is connected to the body 200.

The object may be a living body of a human or animal, tissues in the living body, such as blood vessels, bones, muscles, etc., but is not limited thereto. Therefore, anything having an internal structure capable of being imaged by the ultrasound imaging apparatus 10 may become the object.

Referring to FIGS. 3A to 3C, the probe 110 may be provided in a linear probe having a surface shape in a linear manner, as illustrated in FIG. 3A, a convex probe having a surface shape in a convex manner, as illustrated in FIG. 3B, or a matrix probe having a surface shape in a matrix manner, as illustrated in FIG. 3C, but is not limited thereto. The probe 110 may have a shape as-well known in the art as well as the probe 110 as illustrated in FIGS. 3A to 3C, such as a prove having a phased array and the likes.

One end of the cable 120 may be connected to the probe 110, and the other end of the cable 120 may be connected to the probe connector 130.

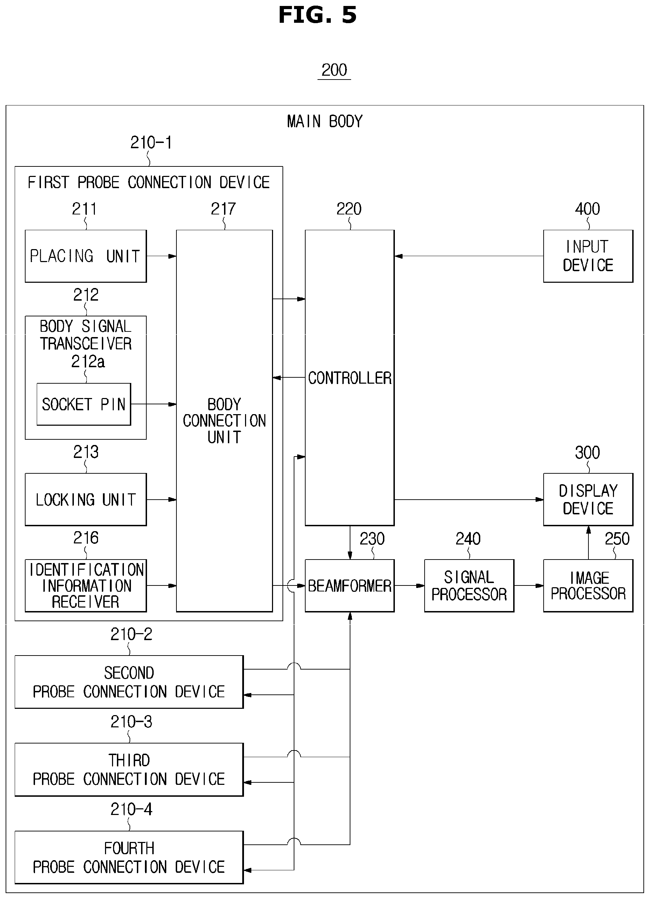

The probe connector 130 may be placed in the probe connection device 210 (refer to FIG. 5) placed in the body 200. After transmitting identification information of the probe assembly, the probe connector 130 may transmit an ultrasonic signal generated by the probe 110 to the probe connection device 210 or may receive a control signal generated by the body 200 by a user's operation.

The probe connector 130 may be realized in a shape of an external connector coupled to the probe connection device 210 realized in a shape of an internal connector.

In addition, referring to FIG. 4, the probe connector 130 according to one embodiment includes a probe signal transceiver 132, a printed circuit board 135, a storage unit 136, and an identification information transmitter 137. In addition, the probe connector 130 may further include a locking shaft 133 so that the probe connector 130 is placed in and locked into the probe connection device 210, but components for the placing and locking is not limited to the locking shaft 133, or the locking shaft 133 may be omitted.

The locking may represent an operation of performing connection between the probe connector 130 and the body 200 through a mechanical coupling, or an operation of controlling the connection in a state where the probe connector 130 and the body 200 are already coupled to each other. The locking is different from that the probe connector 130 is simply and mechanically placed in the body 200.

The locking includes a first locking to connect the identification information transmitter 137 to an identification information receiver 216 of the probe connection device 210 and a second locking to connect the probe signal transceiver 132 of the probe connector 130 to a body signal transceiver 212 of the probe connection device 210. Here, the connection includes contact type connection and non-contact type connection.

Each of the first locking and the second locking may be performed by a user's manual operation or may be performed automatically. For example, when the probe connection device 210 includes a separated locking unit 213, the first locking and the second locking may be performed by driving of the locking unit 213, a detail description thereof will be described later.

The storage unit 136, which is a storage medium storing identification information, may include an active electronic tag in which own battery is embedded, and a passive electronic tag operated by radio waves from the identification information transmitter 137 as a power, and maybe realized by various storage media, Magnetic Storage medium, memory semiconductor, flash memory, and the like.

The identification information includes all information related to the probe assembly 100, and probe assembly identification information such as probe model information, version information, and serial number.

The first probe assembly 100-1 to the fourth probe assembly 100-4 may have identification information different from each other, and the body 200 may distinguish from the first probe assembly 100-1 to the fourth probe assembly 100-4 according to the identification information.

The storage unit 136 may be realized by a portable storage medium.

When the first locking is performed, the recognition transmitter 137 may be transmission media, particularly, the recognition transmitter 137 may transmit identification information stored in the storage unit 136 to the controller 220 through the identification information receiver 216 of the body 200.

The first locking and mechanically placing may be performed at the same time. In this case, the identification information transmitter 137 may transmit the identification information to the identification information receiver 216 in a state where the probe connector 130 is placed in the probe connection device 210.

A detail description of the identification information transmitter 137 will be described later.

When the second locking is performed, the probe signal transceiver 132 may receive a control signal from the body signal transceiver 212 (refer to FIG. 5) of the body 200, or may transmit an ultrasonic signal generated by the probe 110 to the body signal transceiver 212 of the body 200.

The probe signal transceiver 132 of the probe connector 130 may be realized by a terminal 132 provided with a conductive connection pin 132a so that a control signal or an ultrasonic signal may be transmitted or received in a contact manner, or may be realized by a wireless communication module so that a control signal or an ultrasonic signal may be transmitted or received in a non-contact manner. Hereinafter the probe signal transceiver 132 realized by a terminal will be described.

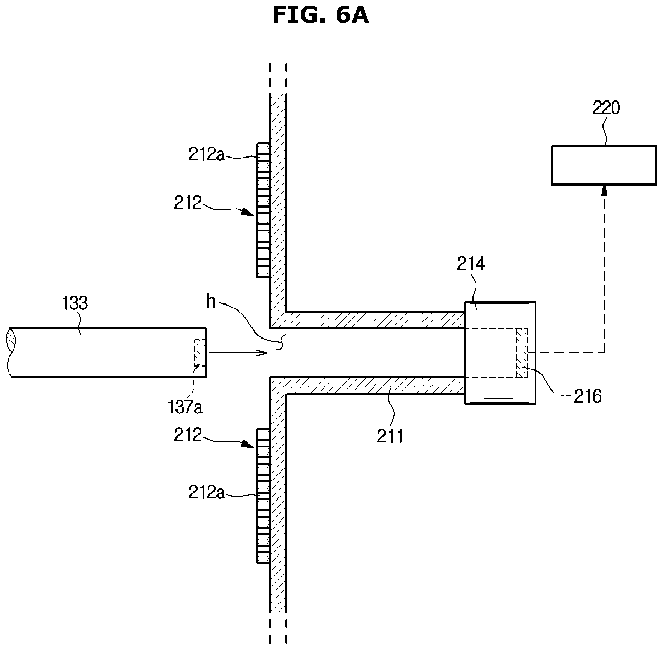

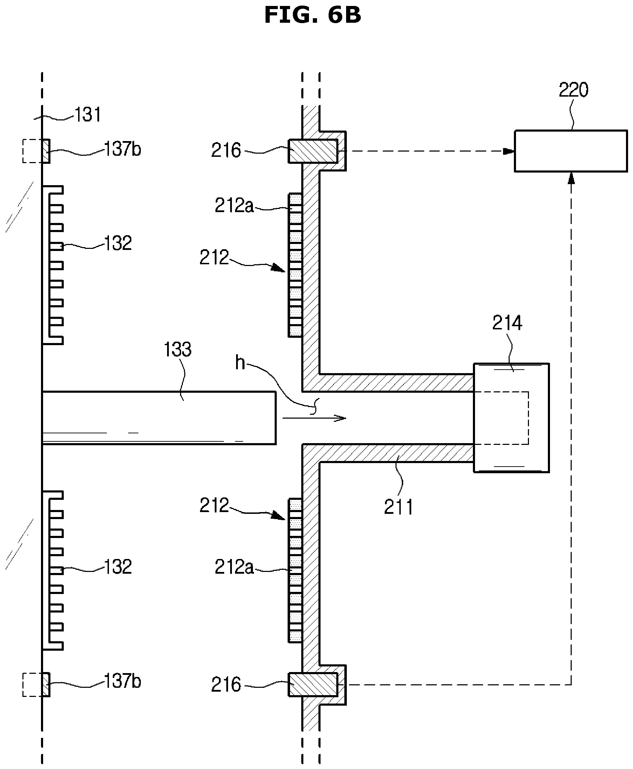

The terminal 132 may include the connection pin 132a inserted into a socket (refer to FIG. 6) of the body 200. When the second locking is performed, the connection pin 132a may transmit an ultrasonic signal to or receive a control signal from a socket pin 212a (refer to FIG. 5) by making contact with the socket pin 212a of the body 200.

When the second locking is performed, the printed circuit board (PCB) 135 may receive a control signal from the terminal 132, and may drive the probe assembly 100 based on the received control signal.

In addition, when the second locking is performed, the PCB 135 may transmit an ultrasonic signal generated by the probe 110 to the terminal 132.

FIG. 5 is a block diagram illustrating a body in accordance with one embodiment of the present disclosure.

A body 200 includes a probe connection 210, a controller 220, a beamformer 230, a signal processor 240, an image processor 250, a display device 300, and an input device 400.

FIG. 5 illustrates that four probe connection devices 210-1 to 210-4 are provided, but the number of the probe connection device 210 may vary depending on the number of a probe assembly 100 provided in the body 200, such as two probe assemblies, three probe assemblies, or five probe assemblies, but is not limited to as illustrated in FIG. 5.

For this, each of the first probe connection device 210-1 to the fourth probe connection device 210-4 include a placing unit 211, a body signal transceiver 212, and an identification information receiver 216. In addition, each of the first probe connection device 210-1 to the fourth probe connection device 210-4 may further include a locking unit 213 to perform automatically a first locking and a second locking, but when the first locking and the second locking are manually performed, the locking unit 213 may be omitted.

Hereinafter the placing unit 211, the body signal transceiver 212, the locking unit 213, and the identification information receiver 216, all of which are included in the first probe connection device 210-1, will be described as an example.

A probe connector 130 is mechanically placed in the placing unit 211.

For example, the placing unit 211 may be realized in various shape configured to allow the probe connector 130 to be placed, such as a housing provided on a front surface of the body 200, or a coupling groove forming member in which a coupling groove h is provided inside the body 200 to allow a locking shaft to be inserted. Hereinafter for convenience of explanation, the placing unit 121 realized by the coupling groove forming member will be described as an example.

When the first locking between the probe connector 130 and the probe connection device 210 is performed, the identification information receiver 216 may receive identification information of the probe assembly 100 from the identification information transmitter 137.

The first locking and mechanically placing may be performed at the same time. In this case, the identification information receiver 216 may be provided in the placing unit 211 so that the identification information receiver 216 may receive identification information by being connected to the identification information transmitter 137 in a state where the probe connector 130 is placed in the probe connection device 210.

A detail description of the identification information receiver 216 will be described later.

When the second locking is performed, the body signal transceiver 212 may receive an ultrasonic signal from the probe signal transceiver 132 of the probe connector 130 or may transmit a control signal generated by the controller 220 to the probe signal transceiver 132 of the probe connector 130.

The body signal transceiver 212 may be realized by a socket 212 provided with a conductive connection pin 132a so that a control signal or an ultrasonic signal may be transmitted or received in a contact manner or may be realized by a wireless communication module so that a control signal or an ultrasonic signal may be transmitted or received to or from the probe signal transceiver 132 of the probe connector 130 in a non-contact manner. Hereinafter the body signal transceiver 212 realized by a socket will be described.

The socket 212 may include a socket pin 212a in which the connection pint 132a of the probe connector 130 is inserted to be connected thereto. When the second locking between the probe connector 130 and the probe connection device 210 is performed, the socket pint 212a may transmit or receive an ultrasonic signal or a control signal to or from the connection pin 132a by making contact with the connection pin 132a of the probe connector 130.

That is, the socket pin 212a electrically connects the body 200 to the probe assembly 100 to allow an ultrasonic signal of the probe assembly 100 to be transmitted to the controller 220 or the beamformer 230, or to allow a control signal of the body 200 to be transmitted to the probe assembly 100.

In order that the probe connector 130 and the body 200 perform connection or disconnection through mechanical coupling, the locking unit 213 may be a component to perform automatic locking and automatic unlocking between the probe connector 130 and the probe connection device 210. The locking and unlocking may represent locking or unlocking of at least one of the first locking or the second locking.

Meanwhile, as above-described embodiment, the probe connection device 210 is described to be realized by a component of the body 200 not an additional device separated from the body 200, but the probe connection device 210 may be configured to be mechanically detachable to the body 200 by being realized by a separated physical device.

In this case, the probe connection device 210 may further include a body connection unit 217.

The body connection unit 217 may perform a function of path connecting each components of the probe connection device 210 to each of components of the body 200, particularly, the body connection unit 217 may transmit identification information, which is received from the probe connector 130 by the identification information receiver 216 of the probe connection device 210, to the body controller 220, or may transmit a control signal of the controller 220 to the socket 212 or a motor of the probe connection device 210

The body connection unit 217 may be realized by a terminal type so that the body connection unit 217 may be connected to the controller 220 by making contact with a separated terminal provided on the body 200, or may be realized by a separated wired/wireless communication module so that the body connection unit 217 may be connected to a wired/wireless communication module (not shown) provided on the body 200.

Meanwhile, when the probe connection device 210 is embedded in the body 200, the body connection unit 217 may be omitted or the body signal transceiver 212 and the identification information receiver 216 of the probe connection device 210 may be directly connected to components of the body 200, such as the controller 220, and the beamformer 230.

The controller 220 generates a control signal, which is configured to control entire components constituting the ultrasound imaging apparatus 10, such as the probe connection device 210, the beamformer 230, the signal processor 240, the image processor 250, the display device 300, the probe assembly 100, and the like, and transmits the control signal to the each component.

According to one embodiment, the first probe connector 130-1 to the fourth probe connector 130-4 are placed, and the first locking between the first probe connector 130-1 to the fourth probe connector 130-4 and a first probe connection device 210-1 to a fourth connection device 210-4 corresponding to the first probe connector 130-1 to the fourth probe connector 130-4, respectively, is performed.

When identification information of the first probe assembly 100-1 to the fourth probe assembly 100-4 is transmitted to the identification information receiver 216 of the body 200 by the first locking, the controller 220 may receive the identification information of the first probe assembly 100-1 to the fourth probe assembly 100-4from each identification information receiver 216.

The controller 220 allows that the identification information of the first probe assembly 100-1 to the fourth probe assembly 100-4 is displayed on the display device 300, and generates a selection signal by receiving a selection command among the first probe assembly 100-1 to the fourth probe assembly 100-4 from a user through the input device 400.

Subsequently, when the probe connection device 210 corresponding to the selected probe assembly 100 and the probe connector 130 perform the second locking, the controller 220 may transmit a control signal to the body signal transceiver 212 so that the body signal transceiver 212 transmits a control signal controlling the probe assembly 100 to the probe signal transceiver 132 or so that an ultrasonic signal received from the probe 100 is received through the body signal transceiver 212.

That is, by the second locking, each component of the selected probe assembly 100 may be electrically connected to components of the body 200, and the body 200 controls the components of the selected probe assembly 100 so that the selected probe assembly 100 may be activated and the ultrasonic signal acquired by the selected probe assembly 100 may be processed or displayed.

In this case, the first locking between the probe connector 130 of the probe assembly 100, which is not selected, and the probe connection device 210 may be released.

Meanwhile, when the probe connection device 210 further includes the locking unit 213, the controller 220 may control the locking unit 213 that the locking unit 213 performs or releases the first locking or the second locking according to the control signal by transmitting a control signal to the locking unit 213.

According to another embodiment, a first probe connector 130-1 to a fourth probe connector 130-4 are placed, and the first locking is performed between a first probe connector 130-1 and a probe connection device 210-1.

When identification information received from the first probe assembly 100-1 is transmitted to the identification information receiver 216 by performing the first locking, the controller 220 may receive the identification information of the first probe assembly 100-1 from the identification information receiver 216.

Subsequently, the controller 220 transmits a control signal to the display device 300 so that the identification information of the first probe assembly 100-1 is displayed through the display device 300, and generates a selection signal by receiving whether to select the first probe assembly 100-1 from a user through the input device 400.

When the user selects the first probe assembly 100-1, the first probe connector 130-1 and the first probe connection device 210-1 performs the second locking, and the controller 220 receives an ultrasonic signal from the first probe connection device 210-1 in which the second locking is performed.

However, when the user does not select the first probe assembly 100-1, the first probe connector 130-1 and the first probe connection device 210-1 releases the first locking, the second probe connector 130-2 and the second probe connection device 210-2 performs the first locking, and thus the controller 220 receives identification information of the second probe assembly 100-2.

Subsequently, the controller 220 allows that identification information of the second probe assembly 100-2 is displayed through the display device 300, and generates a selection signal by receiving whether to select the second probe assembly 100-2 from a user through the input device 400.

When the user selects the second probe assembly 100-2, the second probe connector 130-2 and the second probe connection device 210-2 performs the second locking, and the controller 220 receives an ultrasonic signal from the second probe assembly 100-2

However, when the user does not select the second probe assembly 100-2, the second probe connector 130-2 and the second probe connection device 210-2 releases the first locking, the third probe connector 130-3 and the third probe connection device 210-3 performs the first locking, and thus the controller 220 receives identification information of the third probe assembly 100-3.

Subsequently, the controller 220 allows that identification information of the third probe assembly 100-3 is displayed through the display device 300, and generates a selection signal by receiving whether to select the third probe assembly 100-3 from a user through the input device 400.

When the user selects the third probe assembly 100-3, the third probe connector 130-3 and the third probe connection device 210-3 performs the second locking, and the controller 220 receives an ultrasonic signal from the third probe assembly 100-3.

However, when the user does not select the third probe assembly 100-3, the third probe connector 130-3 and the third probe connection device 210-3 releases the first locking, the fourth probe connector 130-4 and the fourth probe connection device 210-4 performs the first locking, and thus the controller 220 receives identification information of the fourth probe assembly 100-4.

Subsequently, the controller 220 allows that identification information of the fourth probe assembly 100-4 is displayed through the display device 300, and generates a selection signal by receiving whether to select the fourth probe assembly 100-4 from a user through the input device 400.

When the user selects the fourth probe assembly 100-4, the fourth probe connector 130-4 and the fourth probe connection device 210-4 performs the second locking, and the controller 220 receives an ultrasonic signal from the probe assembly 100-4.

However, when the user does not select the fourth probe assembly 100-4, the fourth probe connector 130-4 and the fourth probe connection device 210-4 releases the first locking, the first probe connector 130-1 and the first probe connection device 210-1 performs the first locking again, and thus the controller 220 receives identification information of the first probe assembly 100-1.

According to as mentioned embodiment, the controller 220 may acquire identification information of each probe assembly 100-1 to 100-4 in order.

When the probe connection device 210 further includes the locking unit 213, the controller 220 may transmit a control signal to the locking unit 213 so that the locking unit 213 may perform or release the first locking or the second locking according to the control signal.

The control unit 220 may include a processor, a ROM in which control programs for control of the ultrasound imaging apparatus 10 are stored, and a RAM in which signals or ultrasound image data inputted from the probe assembly 100 of the ultrasound imaging apparatus 10 or the input device 400 are stored or which is used as a storage area to correspond to various operations performed in the ultrasound imaging apparatus 10.

A graphic processing board in which a processor, and RAM or ROM are provide on a separated circuit board electrically connected to the controller 220 may be included. The process, a ROM, and RAM may be connected to each other through an internal bus.

In addition, the controller 220 may be used as a term representing a component including a processor, a RAM, and a ROM.

The controller 220 may be used as a term representing a component including a processor, a RAM, a ROM and a processing board.

The beamformer 230 converts an ultrasonic signal received from any one of the first probe assembly 100-1 to the fourth probe assembly 100-4 into an analog/digital, performs proper time-delay on the converted reception signal, accumulates the time-delayed reception signal, and outputs digital reception focused beam indicating a level of energy reflected from a focal point on a transmission scan line.

The signal processor 240 filters noise in the digital reception focused beam to improve an ultrasound image quality, and performs envelope detection processing configured to detect the strength of the reception signal based on the filtered reception focused beam so that digital ultrasound image data is generated.

The image processor 250 performs scan conversion to convert scan line of the digital ultrasound image data so that the digital ultrasound image data may be displayed on a display region of the display device 300, and performs image processing, such as B-mode image processing, and Doppler image processing, on the digital ultrasound image data so that an ultrasound image in a user's desired shape based on the scan converted digital ultrasound image data may be displayed on the display device 300.

The image processor 250 performs RGB processing on the ultrasound image data and transmits the processed ultrasound image data to the display device 300 so that an ultrasound image, which is processed from the digital ultrasound image data, may be displayed on the display device 300.

The display device 300 displays identification information received from the plurality of probe connectors 130-1 to 130-4 placed in the plurality of probe connection devices 210-1 to 210-4, and displays an ultrasound image generated from digital ultrasound image data received from the probe connector 130 of any one probe assembly 100, which is selected through the input device 300,

A plurality of the display device 300 may be provided in the ultrasound imaging apparatus 10.

The input device 400 receives a certain instruction and command from a user to control the ultrasound imaging apparatus 10. The input device 400 may include a user interface, such as a keyboard, a mouse, a trackball, a touch screen, or a paddle.

The input device 400 may include a separated button to receive selection command of any probe assembly 100 from the first probe assembly 100-1 to the fourth probe assembly 100-4, all of which are placed.

Hereinafter referring to FIGS. 6A to 7, a process of transmitting identification information of any probe assembly 100 among the first probe assembly 100-1 to the fourth probe assembly 100-4 to the body 200 will be described in detail.

FIGS. 6A to 7 are views illustrating an identification information transmitter and an identification information receiver in accordance with one embodiment of the present disclosure.

Hereinafter the first locking and the second locking will be described as the first locking is manually performed by a user's operation and the second locking between the probe connector 130 and the probe connection device 210 is automatically performed according to a control signal of the controller 220 since the probe connection device 210 includes the locking unit 213, and the locking unit 213 includes a coupling member 214 and a motor 215.

Alternatively, the first locking and the second locking may be performed as the first locking is automatically performed since the probe connection device 210 includes the locking unit 213, and the second locking is manually performed by a user's operation, or as the first locking and the second locking are performed by manual operation or performed by the locking unit 213, but is not limited thereto.

In addition, the locking unit 213 is not limited to be realized as mentioned above, and may be realized in various shapes such as electromagnet, external screw, internal screw, for mechanical coupling.

Referring to FIGS. 6A and 6B, the coupling member 214 may be provided in a point where the coupling groove h is extended.

An end portion of the locking shaft 133 inserted into the coupling groove h may be placed in the coupling member 214.

The identification information transmitter 137 may be realized by a conductive contact mount (hereinafter refer to as a first contact mount 137a and 137b), and in this case, the identification information receiver 216 also may be realized by a second contact mount 216 configured to make contact with the first contact mount 137a and 13b by coupling to the probe connector 210.

As illustrated in FIG. 6A, the first contact mount 137a may be provided in the end portion of the locking shaft 133, and the end portion of the locking shaft 133 is placed in the coupling member 214 by a user so that the first locking may be performed.

By performing the first locking, the first contact mount 137a provided in the end portion may transmit identification information of the probe assembly 100 to the second contact mount 216 provided in the coupling member 214 of the probe connection device 210.

As illustrated in FIG. 6B, the first contact mount 137b may be provided in a rear surface of a housing 131 of the probe connector 130, and the locking shaft 133 is inserted into the coupling groove h by a user so that the first contact mount 137a makes contact with the second contact mount 216, and thus the first locking may be performed.

By performing the first locking, the first contact mount 137b provided in the rear surface may transmit identification information to the second contact mount 216 provided in a front surface of the probe connection device 210.

The first contact mount 137b and the second contact mount 216 may be provided in plural to transmit precisely identification information.

As illustrated in FIGS. 6A and 6B, a user may manually operate to perform the first locking, and also the probe connector 130 or the probe connection device 210 are provided with a motor or actuator so that the first locking may be performed according to a control signal of the controller 220.

In a state where the first contact mount 137a and 137b and the second contact mount 216 makes contact with each other in advance, the controller 220 transmits a control signal to the identification information receiver 216 so that the identification information receiver 216 receives identification information, and thus the first locking between the probe connection device 210 and the probe connector 130 may be performed.

The position of the first contact mount 137a and the second contact mount 216 are not limited to the position as illustrated in FIGS. 6A and 6B, and may be placed at any position in the probe connector 130 and the probe connection device 210.

The motor 215 rotates the locking shaft 133 placed in the coupling member 214 by applying a torque to the coupling member 214 according to a control signal of the controller 220, and thus the probe connector 130 is mechanically coupled to the probe connection device 210 so that the second locking is performed.

By performing the second locking between the probe connector 130 and the probe connection device 210, the socket pin 212a may be connected to the connection pin 132a.

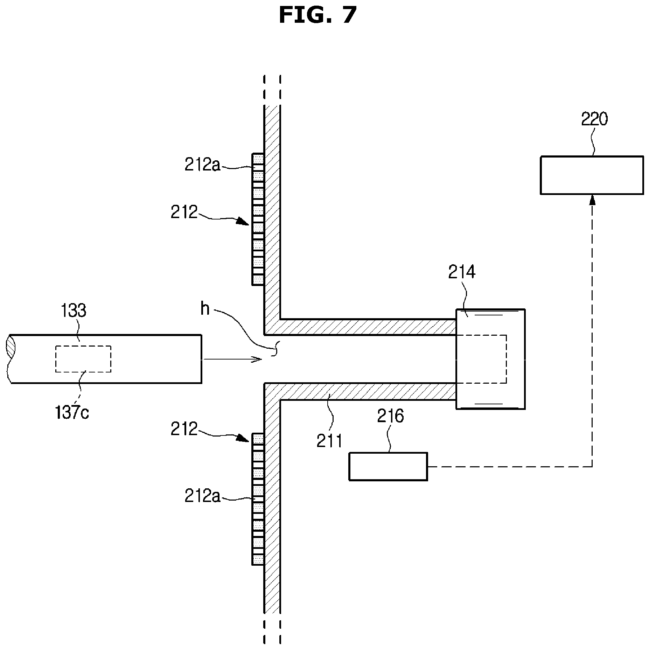

Referring to FIG. 7, the identification information transmitter 137 may be realized by a wireless communication module 137c or an antenna, and may be communicated with the identification information receiver 216 through a wireless network, wherein the identification information receiver 216 realized by a wireless communication module corresponding to a communication method of the identification information transmitter 137.

In this case, the wireless network may include a wireless communication network, and a local area network (LAN), and a combination of a wireless communication network and a local area network.

In a location where access point (AP) is installed, a wireless communication network may be connected to access point (AP) by connecting to a wireless network. A wireless communication network supports wireless LAN standard (IEEE802. 11x) of the Institute of Electrical and Electronics Engineers (IEEE).

A local area network includes a Bluetooth communication network, Bluetooth Low Energy, infrared data association (IrDA), Wi-Fi network, Ultra Wideband (UWB), RFID networks, Near Field Communication (NFC) and Zigbee communication network and the like.

The first locking between the probe connection device 210 and the probe connector 130 includes manual operation by a user or performing automatic locking by the locking unit 213 so that the identification information transmitter 137 and the identification information receiver 216 are disposed within a certain range to be connected to each other.

In a state where the identification information transmitter 137 and the identification information receiver 216 are disposed within a certain range to be connected to each other, the first locking between the probe connection device 210 and the probe connector 130 may include that the controller 220 transmits a control signal to the identification information receiver 216 to allow the identification information receiver 216 to receive identification information.

The identification information transmitter 137 and the identification information receiver 216 may be realized by a wireless communication module corresponding to a wireless network connected to each other.

In this case, a voltage may be supplied to the probe assembly 100 according to the used wireless network, and for this, the probe assembly 100 may further include power source (not shown). In addition, the probe assembly 100 may be supplied a voltage from the power source (not shown) of the body 200.

As mentioned above, the identification information transmitter 137 provided in the probe connector 210 transmits identification information to the identification information receiver 216 provided in the probe connection device 210 through the first locking so that the identification information may be transmitted to the controller 220 of the body 200.

In this case, the position of the identification information transmitter 137 realized by the wireless communication module 137c or the antenna is not limited to the position as illustrated in FIG. 7, and may be placed in any position in the probe connector 130 and the probe connection device 210.

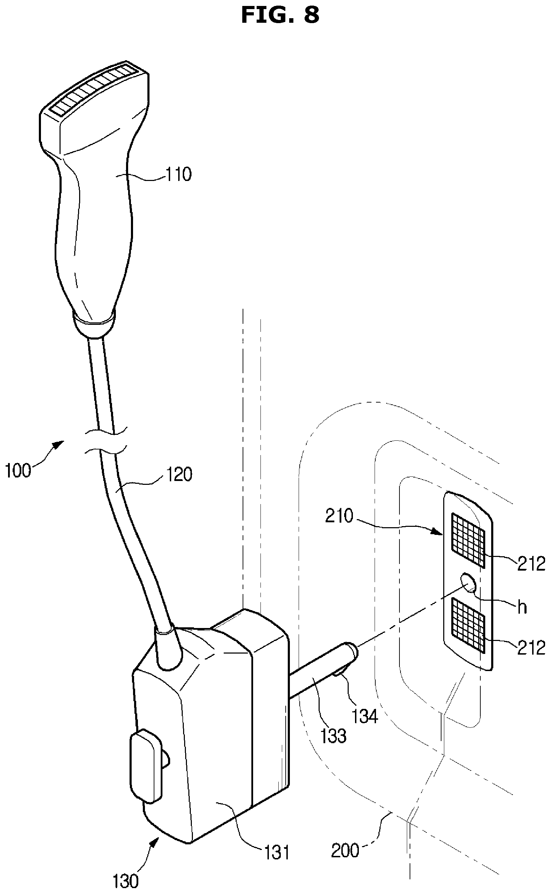

Hereinafter a process of performing the first locking and the second locking between the probe connector 130 of any probe assembly 100 among the first probe assembly 100-1 to the fourth probe assembly 100-4, and the probe connection device 210 to which the probe connector 130 is placed, will be described in detail with reference with FIGS. 8 to 11c.

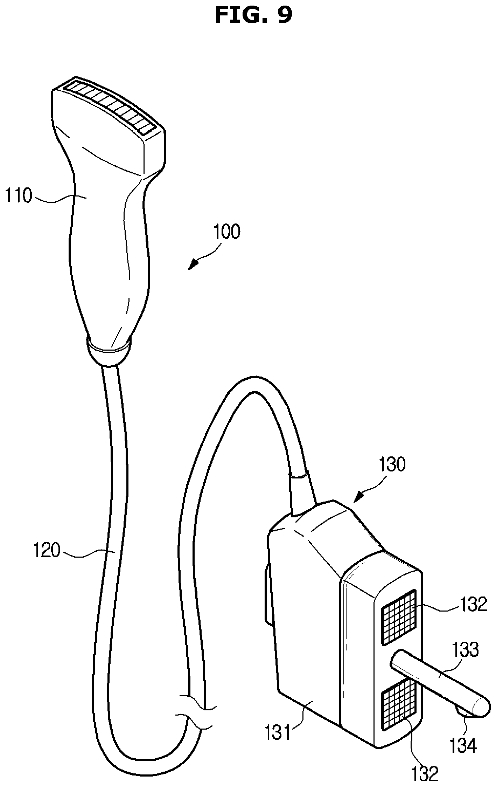

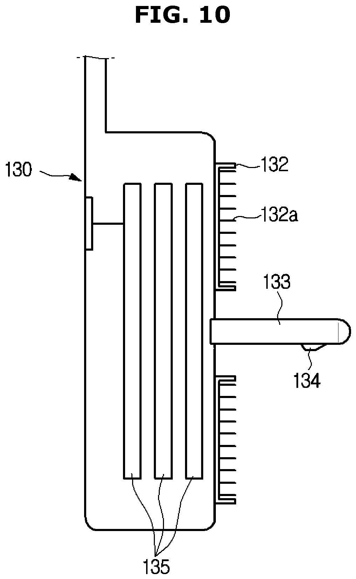

FIG. 8 is an external perspective view from front of a probe connector and a probe connection device, FIG. 9 is an external perspective view from back of a probe connector, and FIG. 10 is an external perspective view from side of a probe connector.

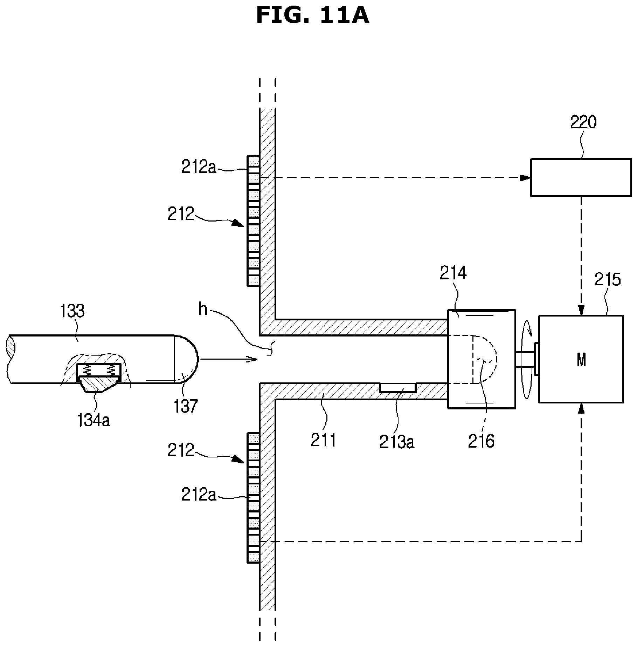

FIGS. 11A to 11C are side views of a locking shaft and a body illustrating a locking method of a probe connector and a probe connection device in accordance with one embodiment of the present disclosure.

Hereinafter for convenience of description, a case where the identification information transmitter 137 of the probe connector 130 is realized by the first contact mount 137a as illustrated in FIG. 6A, and the identification information receiver 216 of the probe connection device 210 is realized by the second contact mount 216 as illustrated in FIG. 6B will be described as an example.

Referring to FIGS. 8 to 10, the probe connector 130 may further include a housing 131, and a locking member 134 performing the second locking between the probe connector 130 and the probe connection device 210. In this case, the locking shaft 133 may be formed in a shape having a cross-section a polygon or an oval for stable fixation with the coupling member 214 of the probe connection device 210.

A user puts the locking shaft 133 into the coupling groove h of the probe connection device 210 so that the probe connector 130 may be placed in the probe connection device 210. In this case, the terminal 132 of the probe connector 130 may be inserted into the socket 212 of the body 200.

However, the probe connector 130 is not connected to the probe connection device 210 by a mechanical "placing", and the first locking and the second locking are performed in order that the probe connector 130 and the probe connection device 210 is connected to each other.

Referring to FIGS. 11A to 11C, a user inserts the locking shaft 133 into the coupling groove h so that an end portion of the locking shaft 133 is coupled to the coupling member 214. Accordingly, the first locking may be performed. The identification information transmitter 137 and the identification information receiver 216 may be electrically connected to each other by making contact with each other through the first locking.

The motor 215 rotates the locking shaft 133 coupled to the coupling member 214 so that the locking member 134 is coupled to the locking groove 213a of the probe connection device 210. Accordingly, the second locking may be performed. The connection pin 132a of the probe connector 130 and the socket pint 212a of the probe connection device 210 may be electrically connected to each other by making contact with each other through the second locking.

Referring to FIG. 11A, the locking shaft 133 according to one embodiment may include the locking member 134 in a shape of the locking pin 134a realized by a structure configured to be inserted into the inside of the locking shaft 133 by external, i.e., an outer wall of the coupling groove h, pressure.

For the second locking between the probe connector 130 and probe connection device 210, when the motor 215 applies a torque to the coupling member 214, the locking shaft 133 is rotated. Accordingly, the locking pin 134a protrudes toward the outside of the locking shaft 133 in the locking groove 213a and is inserted into the locking groove 213a. When the locking pin 134a is inserted into the locking groove 213a, the connection pin 132a of the probe connector 130 is moved, and the connection pin 132a and a socket pin (not shown) of the socket 212 inside the body may be electrically connected to each other by making contact with each other. Accordingly, the second locking between the probe connection device 210 and the probe connector 130 is completed.

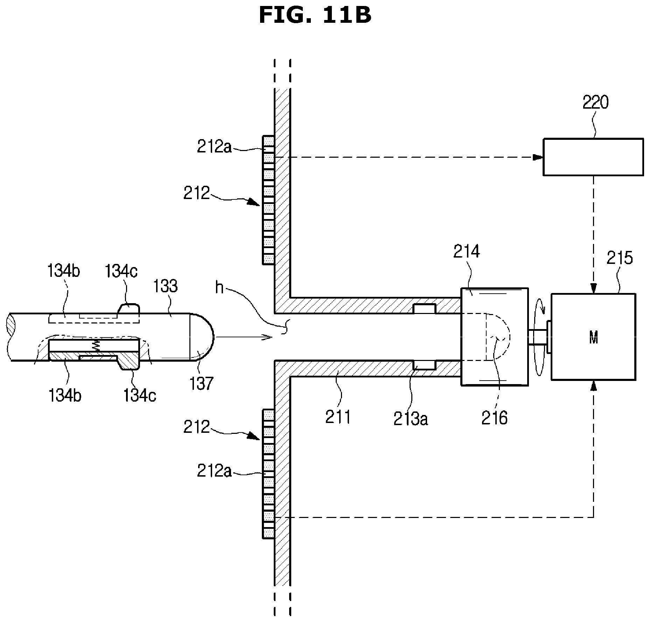

Referring to FIG. 11B, a locking shaft 133 according to another embodiment include a locking member 134 realized by a pressure unit 134b and a locking pin 134c connected to the pressure unit 134b so that the locking shaft 133 may be realized by a structure in which the locking pin 134c is inserted to the inside of the locking shaft 133 when a user presses the pressure unit 134b. The pressure unit 134b and the locking pin 134c may be provided in plural.

For the first locking between the probe connector 130 and the probe connection device 210, a user may insert the locking shaft 133 into the coupling groove h after pressing the pressure unit 134b,and may place an end portion of the locking shaft 213 into the coupling member 214.

As the same as FIG. 11A, for the second locking between the probe connector 130 and probe connection device 210, when the motor 215 applies a torque to the coupling member 214, the locking shaft 133 is rotated and the locking pin 134a is inserted into the locking groove 213a. When the locking pin 134a is inserted into the locking groove 213a, the connection pin 132a of the probe connector 130 is moved, and the connection pin 132a and a socket pin 212a of the socket 212 inside the body may be electrically connected to each other by making contact with each other. Accordingly, the second locking between the probe connection device 210 and the probe connector 130 is completed.

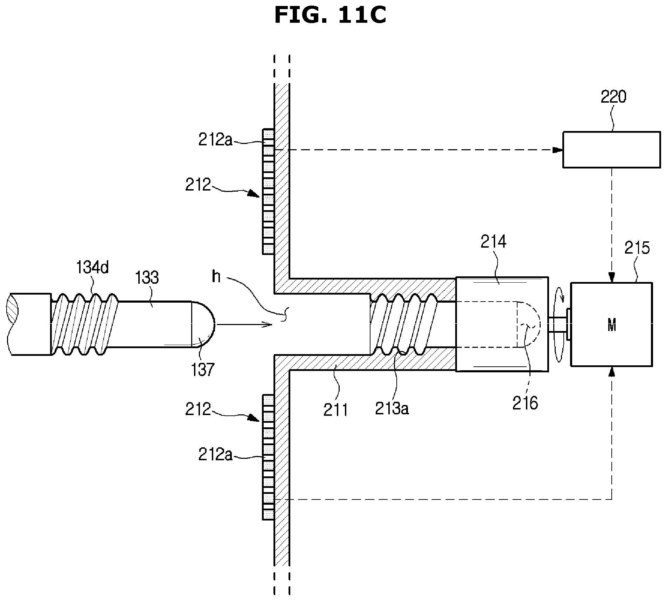

Referring to FIG. 11C, a locking shaft 133 according to another embodiment may include a locking member 134 realized by a rotation coupling unit 134d. In this case, the rotation coupling unit 134d may be realized by an external screw 134d or bolt 134d both of which have grooves on the outside thereof by forming screw threads and screw grooves, and the locking groove 213 may be realized by an internal screw 213a or a nut 213a both of which have grooves on the inside thereof.

For the first locking between the probe connector 130 and the probe connection device 210, a user may insert the locking shaft 133 into the coupling groove h so that the end portion of the locking shaft 213 is placed in the coupling member 214.

As illustrated in FIGS. 11A and 11B, for the second locking between the probe connector 130 and probe connection device 210, when the motor 215 applies a torque to the coupling member 214, the locking shaft 133 is rotated and the external screw 134d and the internal screw 213a are rotatably coupled to each other. Accordingly, the connection pin 132a of the probe connector 130 is moved, and the connection pin 132a and a socket pin (not shown) of the socket 212 inside the body may be electrically connected to each other by making contact with each other. Accordingly, the second locking between the probe connection device 210 and the probe connector 130 is completed.

Meanwhile, when the first locking is completed, the probe connector 130 and the probe connection device 210 may release the first locking. In this case, the motor 215 may rotate the coupling member 214, to which the locking shaft 133 is coupled, in an opposite direction, or may apply repulsive force to the locking shaft 133 according to a control signal of the controller 220 so that the identification information receiver 216 and the identification information transmitter 137 are spaced apart from to each other.

In the above-described embodiment, performing the first locking and the second locking since the probe connector 130 includes the locking shaft 133 and the locking pin 134a, and the probe connection device 210 includes the locking groove 213a, the coupling member 214, and the motor 215 is described, a locking method of the probe connector 130 and the probe connection device 210 is not limited thereto, and thus the locking method may be realized by various methods, such as a method in which the housing 131 of the probe connector 130 realized by an external screw type is coupled to a housing (not shown) of the probe connection device 210 realized by an internal screw type, a toggle-type coupling method, or a clip-type coupling method.

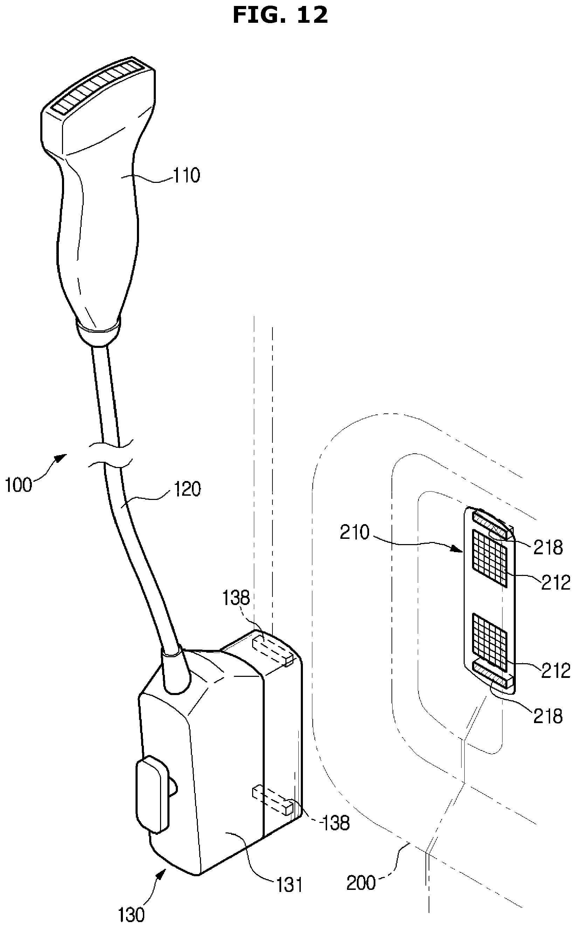

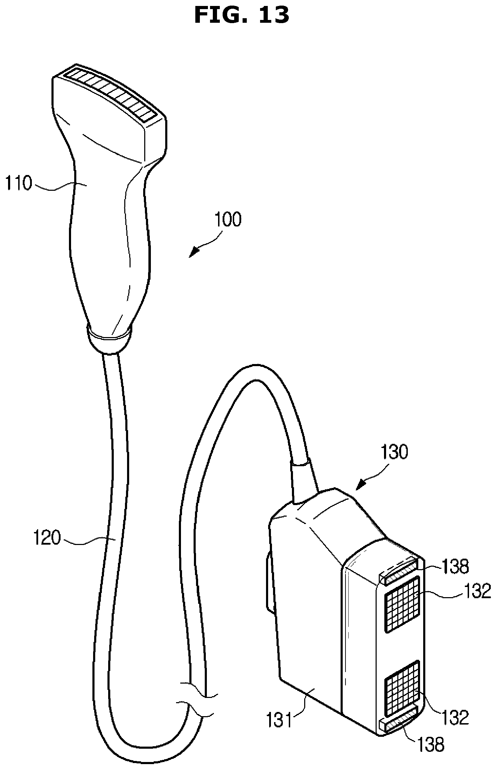

FIGS. 12 and 13 are external views of a probe connector and a probe connection device illustrating a locking method of a probe connector and a probe connection device in accordance with another embodiment of the present disclosure. A probe connector 130 according to another embodiment as illustrated in FIGS. 12 and 13 may include a housing 131, a terminal 132, a printed circuit board 135, a connection pin 132a, a storage unit 136, and a identification information transmitter 137, a description thereof is the same as mentioned above and a duplicate description will be omitted.

A probe connection device 210 according to another embodiment as illustrated in FIGS. 12 and 13 may include a socket 212, an identification information receiver 216, and a body connection unit 217, a description thereof is the same as mentioned above and a duplicate description will be omitted. However, the body connection unit 217 may be omitted as mentioned above.

However, according to another embodiment, an electromagnet 138 instead of the locking shaft 133 may be provided on a rear surface of the probe connector 130, and an electromagnet 218 as a locking unit 213 instead of a coupling groove h and a coupling member 214 may be provided on a front surface of the probe connection device 210.

In this case, the electromagnet 138 provided on the probe connector 130 is attached to the electromagnet 218 provided on the front surface of the probe connection device 210 so that the first locking between the probe connector 130 and the probe connection device 210 may be performed.

Therefore, a user may perform the first locking between the probe connector 130 and the probe connection device 210 by attaching the electromagnet 138 provided on the probe connector 130 to the electromagnet 218 provided on the probe connection device 210, and the identification information transmitter 137 of the probe connector 130 may transmit identification information of the probe assembly 100 stored in the storage unit 136 to the controller 220 of the body 200 through the identification information receiver 216 of the probe connection device 210.

The identification information transmitter 137 and the identification information receiver 216 may be placed any position in the probe connector 130 and the probe connection device 210, and as mentioned above, may be realized by a contact mount or a wireless communication module. The controller 220 of the body 200 may display identification information received from the plurality of probe assemblies 100 on the display device 300 to a user, and may receive whether to select any one probe assembly 100 from a user.

In addition, although not shown, according to another embodiment, a probe connection device 210 may further include a driving device which is provided on a rear surface of an electromagnet 218 and generates gravitation pulling the electromagnet 218 toward a body 200 according to a control signal of a controller 220.

In this case, by the gravitation of the driving device, the electromagnet 218 is moved toward the body 200, and the probe connector 130 coupled to the electromagnet 218 of the probe connection device 210 is also moved toward the body 200. Therefore, the connection pin 132a of the probe connector 130 is moved to make contact with the socket pin 212a provided inside the socket 212 of the probe connection device 210 so that the second locking between the probe connector 130 and the probe connection device 210 may be performed. At this time, the driving device may be realized by a motor or a linear actuator including a piston. Accordingly, in order to perform the second locking with the probe connector 130 of the selected probe assembly 100, the controller 220 transmits a control signal to the motor so that the motor pulls the electromagnet 218 of the probe connection device 210, and the electromagnet 218 is moved toward the body 200 by the gravitation of the motor.

The electromagnet 138 of the probe connector 130 coupled to the electromagnet 218 of the probe connection device 210 is also moved toward the body 200. Accordingly, the terminal 132 of the probe connector 130 is electrically connected to the socket pin of the probe connection device 210 by making contact with the socket pin of the probe connection device 210 so that the second locking between the probe connector 130 and the probe connection device 210 is completed.

In order to release the first locking between the probe connector 130 and the probe connection device 210, the motor may push the electromagnet 218 provided on the front surface of the probe connection device 210 outward the body 200 according to a control signal of the controller 220.

According to another embodiment, all the above-mentioned locking shaft 133 and electromagnet 138 may be proved on a probe connector 130 and a probe connection device 210 so that the first locking or the second locking may be performed, in addition, the first locking or the second locking between the probe connector 130 and the probe connection device 210 may be performed in various methods.

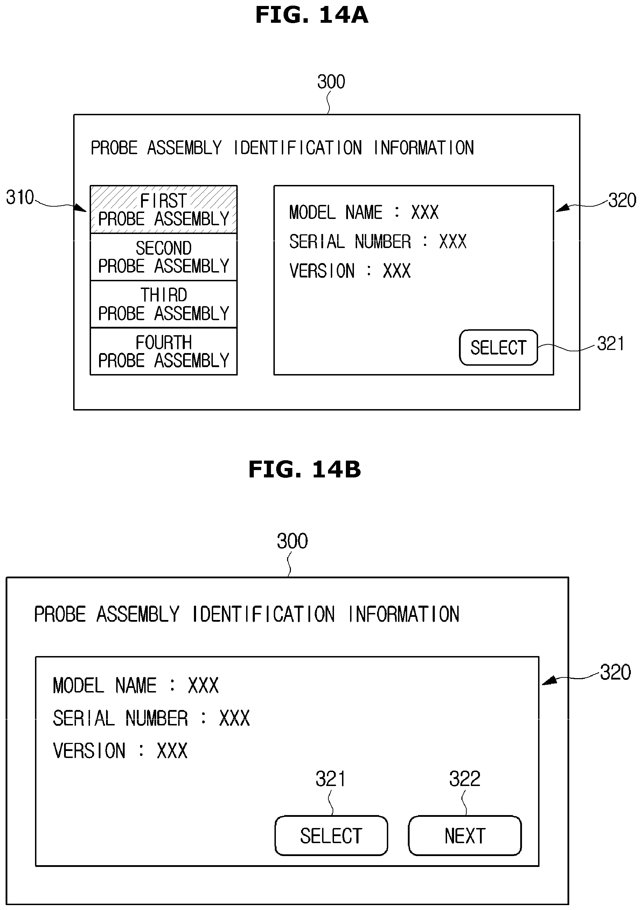

FIG. 14A is an exemplary view illustrating a probe identification information screen displayed by a display device when placed plurality of probe connectors 130 performs the first locking with each probe connection device 210 at the same time.

Referring to FIG. 14A, the display device 300 displays identification information of each probe assembly 100-1 to 100-4 in which the first locking is performed according to a user's operation. For example, when the user selects the first probe assembly 100-1, an identification information screen 320 displaying identification information of the first probe assembly 100-1, such as a model name, a serial number, and a version of the probe assembly 100 may be displayed.

In this case, the user may click a selection button 321 on the identification information screen 320 by operating the input device 400. When the user clicks the selection button 321, the first probe assembly 100-1 may be selected, the second locking between the first probe connector 130-1 and the first probe connection device 210-1 may be performed, and the first locking between the second probe connector 130-2 to the fourth probe connector 130-4 and the second probe connection device 210-2 to the fourth probe connection device 210-4 may be released.

FIG. 14B is an exemplary view illustrating a probe identification information screen displayed by a display device when placed plurality of probe connectors 130 performs the first locking with each probe connection device 210 in sequence.

Referring to FIG. 14B, a display device 300 firstly displays identification information of the first probe assembly 100-1 in which the first locking is performed.

In this case, the user may click the selection button 321 on the identification information screen 320 by operating the input device 400. When the user clicks the selection button 321, the first probe assembly 100-1 may be selected, and the second locking between the first probe connector 130-1 and the first probe connection device 210-1 may be performed.

However, the user may click a next button 322 on the identification information screen 320 by operating the input device 400. When the user clicks the next button 322, the first locking between the first probe connector 130-1 and the first probe connection device 210-1 may be released, the first locking between the second probe connector 130-2 and the second probe connection device 210-2 may be performed, and the display device 300 may display identification information of the second probe assembly 100-2, in which the first locking is performed.

In this case, the user may click the selection button 321 on the identification information screen 320 by operating the input device 400. When the user clicks the selection button 321, the second probe assembly 100-2 may be selected, and the second locking between the second probe connector 130-2 and the second probe connection device 210-2 may be performed.

However, the user may click the next button 322 on the identification information screen 320 by operating the input device 400. When the user clicks the next button 322, the first locking between the second probe connector 130-2 and the second probe connection device 210-2 may be released, the first locking between the third probe connector 130-3 and the third probe connection device 210-3 may be performed, and the display device 300 may display identification information of the third probe assembly 100-3, in which the first locking is performed.

In this case, the user may click the selection button 321 on the identification information screen 320 by operating the input device 400. When the user clicks the selection button 321, the third probe assembly 100-3 may be selected, and the second locking between the third probe connector 130-3 and the third probe connection device 210-3 may be performed.

However, the user may click the next button 322 on the identification information screen 320 by operating the input device 400. When the user clicks the next button 322, the first locking between the third probe connector 130-3 and the third probe connection device 210-3 may be released, the first locking between the fourth probe connector 130-4 and the fourth probe connection device 210-4 may be performed, and the display device 300 may display identification information of the fourth probe assembly 100-4, in which the first locking is performed.

In this case, the user may click the selection button 321 on the identification information screen 320 by operating the input device 400. When the user clicks the selection button 321, the fourth probe assembly 100-4 may be selected, and the second locking between the fourth probe connector 130-4 and the fourth probe connection device 210-4 may be performed.

However, the user may click the next button 322 on the identification information screen 320 by operating the input device 400. When the user clicks the next button 322, the first locking between the fourth probe connector 130-4 and the fourth probe connection device 210-4 may be released, the first locking between the first probe connector 130-1 and the first probe connection device 210-1 may be performed again, and the display device 300 may display identification information of the first probe assembly 100-1, in which the first locking is performed.

The display device 300 is not limited to a screen as illustrated in FIGS. 14A and 14B, and the display device 300 may display identification information of a probe in various ways.

Hereinafter a control method of the above-mentioned ultrasound imaging apparatus will be described.

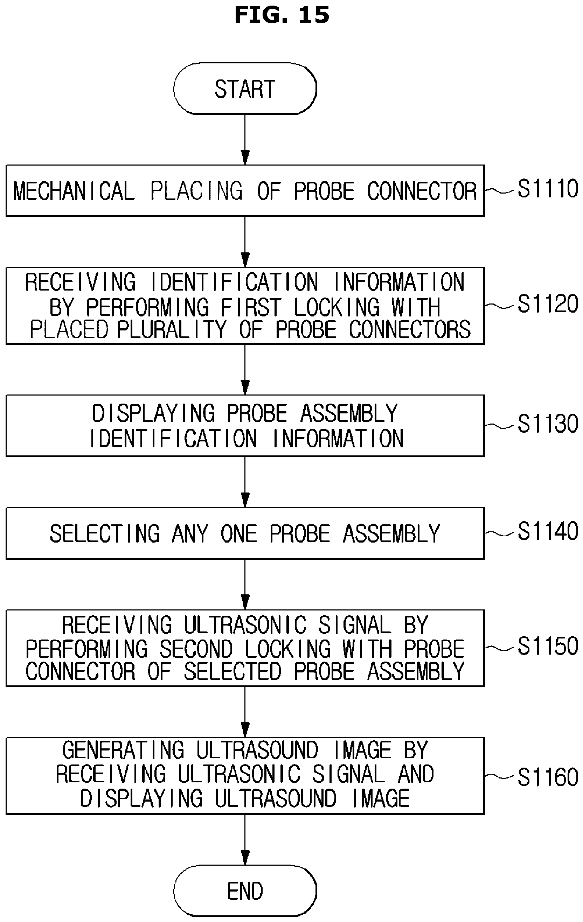

FIG. 15 is a control block diagram of an ultrasound imaging apparatus in one embodiment of the present disclosure.

When the plurality of probe connectors 130-1 to 130-4 is mechanically placed in the plurality of probe connection devices 210-1 to 210-4, respectively S 1110, the first locking between the plurality of probe connectors 130-1 to 130-4 and the plurality of probe connection devices 210-1 to 210-4 may be performed, the identification information transmitter 137 of the plurality of probe connectors 130-1 to 130-4 may transmit identification information, which is specific information of the probe assembly 100, to the identification information receiver 217 of the plurality of probe connection devices 210-1 to 210-4, and thus the identification information may be transmitted to the controller 220 S 1120. The identification information of the probe assembly 100 may be information pre-stored in the storage unit 136 of the plurality of probe assemblies 100-1 to 100-4.

In this case, the identification information transmitter 137 of the plurality of probe connectors 130-1 to 130-4 and the identification information receiver 217 of the plurality of probe connection devices 210-1 to 210-4 may be realized by an electrical contact mount, and a position where of the contact mount is proved is not limited. In addition, the identification information transmitter 137 of the plurality of probe connectors 130-1 to 130-4 and the identification information receiver 217 of the plurality of probe connection devices 210-1 to 210-4 may be realized by a wireless communication module, wherein the communication method, such as, Wi-Fi network, RFID networks, Near Field Communication (NFC) and infrared data association (IrDA) and the like is existed, but is not limited thereto.

The display device 300 may display identification information of each probe 100 assembly received from the plurality of probe assemblies 100-1 to 100-4 to a user S 1130. The identification information may include specific information of the probe assembly 100, such as a model name, a serial number, a version and the like.

A user may select any one probe assembly 100 by operating the input device 400 S 1140, the second locking between the probe connector 130 of the selected probe assembly 100 and the probe connection device 210 corresponding to the probe connector 130 may be performed, and thus the controller 220 or the beamformer 230 may receive an ultrasonic signal generated in the probe assembly 100 through the connection pin 132a of the selected probe 100 S 1150.

The signal processor 240 and the image processor 250 may generate image data and an ultrasound image based on the received ultrasonic signal, and the display device 300 may display the ultrasound image S 1160.

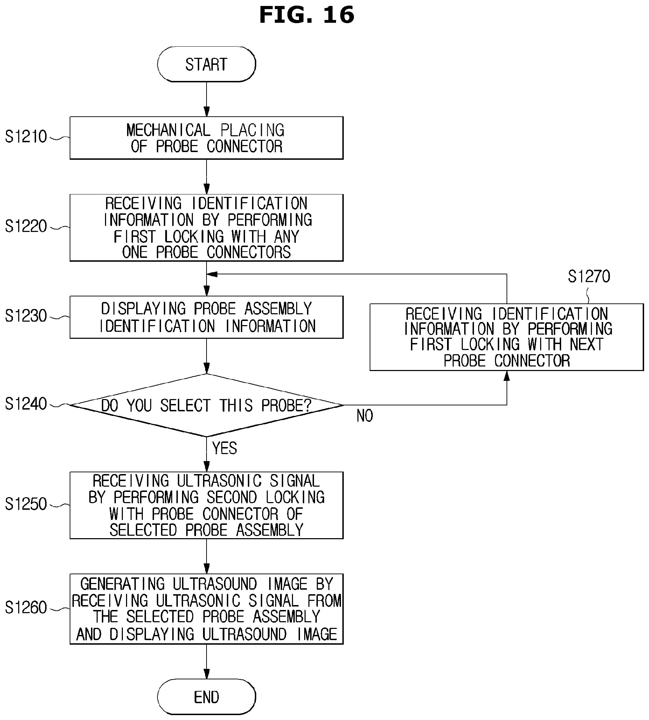

FIG. 16 is a control block diagram of an ultrasound imaging apparatus in another embodiment of the present disclosure.

When the plurality of probe connectors 130-1 to 130-4 is mechanically placed in the plurality of probe connection devices 210-1 to 210-4, respectively S 1210, the first locking between any one probe connector and a probe connection device 210 corresponding to the probe connector may be performed, the identification information transmitter 137 of the probe connector 130, in which the first locking is performed, may transmit identification information, which is specific information of the probe assembly 100, to the identification information receiver 217 of the probe connection device 210, and thus the identification information may be transmitted to the controller 220 S 1220. The identification information of the probe assembly 100 may be information pre-stored in the storage unit 136 of the probe assembly 100.

As mentioned above, the identification information transmitter 137 of the probe connector 130 and the identification information receiver 217 of the probe connection device 210 may be realized by an electrical contact mount, and a position, the contact mount is provided, is not limited. In addition, the identification information transmitter 137 of the probe connector 130 and the identification information receiver 217 of the probe connection device 210 may be realized by a wireless communication module, wherein the communication method, such as, Wi-Fi network, RFID networks, Near Field Communication (NFC) and infrared data association (IrDA) and the like is existed, but is not limited thereto.

The display device 300 may display identification information received from any one probe assembly 100, in which the first locking is performed, to a user S 1230. The identification information may include specific information of the probe assembly 100, such as a model name, a serial number, a version and the like of any one probe assembly 100, in which the first locking is performed.

A user may determine whether to select any one probe assembly 100, in which the first locking is performed, by operating the input device 400 S 1240.

When any one probe assembly 100, in which the first locking is performed, is selected Yes of S 1240, the second locking between the probe connector 130 of the selected probe assembly 100 and the probe connection device 210 may be performed, the controller 220 or the beamformer 230 may receive an ultrasonic signal generated in the probe assembly 100 through the connection pin 132a of the selected probe assembly 100 S 1250.

The signal processor 240 and the image processor 250 may generate image data and an ultrasound image based on the received ultrasonic signal, and the display device 300 may display the ultrasound image S 1260.

When any one probe assembly 100, in which the first locking is performed, is not selected No of S 1240, the first locking between the probe connector 130 of the selected probe assembly 100 and the probe connection device 210 may be released, the first locking between a probe connector 130 of another probe assembly 100 and a probe connection device 210 corresponding to a probe connector 130 may be performed. The identification information transmitter 137 of the probe connector 130, in which the first locking is performed, may transmit identification information, which is specific information of the probe assembly 100, to the identification information receiver 217 of the probe connection device 210, and thus the identification information may be transmitted to the controller 220 S 1270.

Similarly, in this case, the display device 300 may display identification information received from another probe assembly 100, in which the first locking is performed, to a user S 1230, and a user may determine whether to select any one probe assembly 100, in which the first locking, is performed by operating the input device 400 S 1240.

When another probe assembly 100, in which the first locking is performed, is selected Yes of S 1240, the second locking between a probe connector 130 of the selected probe assembly 100 and a probe connection device 210 may be performed, a controller 220 or a beamformer 230 may receive an ultrasonic signal generated in the probe assembly 100 through the connection pin 132a of the selected probe 100 S 1250.

The control method of the ultrasound imaging apparatus 10 may be implemented in a computer-readable medium with computer-readable codes. The computer-readable medium may be any medium that can store data readable by a computer system. For example, the computer-readable medium may be Read Only Memory (ROM), Random Access Memory (RAM), magnetic tape, magnetic disk, flash memory, and optical data storage medium. In addition, the medium may be distributed to computer systems over a network, in which computer-readable code may be stored and executed in a distributed manner.

As is apparent from the above description, according to the proposed a probe assembly, an ultrasound imaging apparatus and a controlling method of the ultrasound imaging apparatus, in a state where the probe assembly is placed in the ultrasound imaging apparatus, the identification information transmitter provided on the probe assembly transmits identification information of the probe assembly to the identification information receiver provided on the ultrasound imaging apparatus so that a user may confirm the identification information of each probe assembly without performing direct electrical connection between the probe signal transceiver of the probe assembly and the body signal transceiver of the ultrasound imaging apparatus.

Although a few embodiments of the present disclosure have been shown and described, it would be appreciated by those skilled in the art that changes may be made in these embodiments without departing from the principles and spirit of the disclosure, the scope of which is defined in the claims and their equivalents.

### DESCRIPTION OF REFERENCE NUMERALS

100: probe assembly
110: probe
111: piezoelectrics
120: cable
130: probe connector
132: probe single transceiver
132a: connection pin
133: locking shaft
135: printed circuit board
136: storage unit
137: identification information transmitter

## Claims

1. An ultrasound imaging apparatus (10) comprising:
a placing unit (211) configured for placing a probe connector (130);
an identification information receiver (216) configured to receive probe assembly identification information from an identification information transmitter (137) of a placed probe connector (130);
an input device (400) configured to receive a selection command of a probe connector (130); and
a locking unit (213) configured to lock the probe connector (130) into the ultrasound imaging apparatus (10) based on the selection command,
wherein the locking unit (213) is configured to perform a first locking connecting the identification information transmitter (137) of the probe connector (130) to the identification information receiver (216) of the ultrasound imaging apparatus (10), and a second locking connecting a probe signal transceiver (132) of the probe connector (130) to a body signal transceiver (212) of the ultrasound imaging apparatus (10), and
wherein the first locking is performed when the probe connector (130) is placed in the placing unit (211) and the second locking is performed when the probe connector (130) is selected.

2. The ultrasound imaging apparatus (10) of claim 1 wherein
the identification information receiver (216) comprises a second contact point receiving the probe assembly identification information by making contact with the identification information transmitter (137) comprising first contact point placed in the probe connector (130).

3. The ultrasound imaging apparatus (10) of claim 2 wherein
the second contact point makes contact with the first contact point by manual operation of a user.

4. The ultrasound imaging apparatus (10) of claim 2 wherein
the locking unit (213) performs the first locking so that the first contact point makes contact with the second contact point.

5. The ultrasound imaging apparatus (10) of claim 1 wherein
the identification information receiver (216) comprises a second wireless communication module receiving the probe assembly identification information by being connected to a first wireless communication module of the identification information transmitter (137).

6. The ultrasound imaging apparatus (10) of claim 1 further comprising:
a display device (300) displaying the probe assembly identification information.

7. The ultrasound imaging apparatus (10) of claim 1 wherein
the locking unit (213) releases the first locking between a probe connector (130), which is not selected from the plurality of probe connectors (130), and the ultrasound imaging apparatus (10).

8. The ultrasound imaging apparatus (10) of claim 1 wherein
when the input device (400) receives whether to select a first probe assembly (100-1), and then the first probe assembly (100-1) is selected, the locking unit (213) performs a second locking between the first probe assembly (100-1) and the ultrasound imaging apparatus (10), and when the first probe assembly (100-1) is not selected, the locking unit (213) performs the first locking between a second probe assembly (100-2) and the ultrasound imaging apparatus (10).

9. The ultrasound imaging apparatus (10) of claim 8 wherein
when the first probe assembly (100-1) is not selected, the locking unit (213) releases the first locking between the first probe assembly (100-1) and the ultrasound imaging apparatus (10).

10. The ultrasound imaging apparatus (10) of claim 1 further comprising:
a controller generating a control signal to control the locking unit (213) wherein the locking unit (213) comprises a motor or an actuator, both of which perform the first locking or the second locking, based on the control signal.

11. The ultrasound imaging apparatus (10) of claim 1 wherein
the placing unit (211) is realized by a coupling groove forming member, in which coupling grooves are formed so that a locking shaft (133) of the probe (110) assembly is placed.

12. The ultrasound imaging apparatus (10) of claim 11 wherein
locking grooves, in which a locking pin of the locking shaft (133) is inserted, are formed in the coupling groove forming member, and the locking unit (213) comprises a coupling member provided at a position formed by an extension of the coupling groove and a motor rotating the locking shaft (133) placed in the coupling member, wherein the motor performs locking by inserting the locking pin into the locking groove by rotating the locking shaft (133).

13. The ultrasound imaging apparatus (10) of claim 1 wherein
the locking unit (213) comprises an electromagnet (218) and a driving device wherein the driving device performs locking by moving the electromagnet (218).

14. A control method of an ultrasound imaging apparatus (10) comprising:
an operation in which an identification information receiver (216) receives probe assembly identification information from an identification information transmitter (137) of a probe connector (130), which is placed in a placing unit (211);
receiving a selection command of a probe connector (130); and an operation in which a locking unit (213) locks the probe connector (130) into an ultrasound imaging apparatus (10) based on the selection command, wherein the operation in which the locking unit (213) locks the probe connector (130) comprises: an operation in which the locking unit (213) performs a first locking connecting the identification information transmitter (137) of the probe connector (130) to the identification information receiver (216) of the ultrasound imaging apparatus (10), and a second locking connecting a probe signal transceiver (132) of the probe connector (130) to a body signal transceiver (212) of the ultrasound imaging apparatus (10), and
wherein the first locking is performed when the probe connector (130) is placed in the placing unit (211) and the second locking is performed when the probe connector (130) is selected.

## Patentansprüche

1. Ultraschallbildgebungsvorrichtung (10), welche folgendes aufweist:
Eine Platzierungseinheit (211), die dafür vorgesehen ist, einen Sondenverbinder (130) zu platzieren;
einen Identifikationsinformationsempfänger (216), der dafür vorgesehen ist, Sondenanordnungsidentifikationsinformationen von einem Identifikationsinformationssender (137) eines platzierten Sondenverbinders (130) zu empfangen; eine Eingabeeinrichtung (400), die dafür vorgesehen ist, einen Auswahlbefehl eines Sondenverbinders (130) zu empfangen; und
eine Verriegelungseinheit (213), die dafür vorgesehen ist, den Sondenverbinder (130) in die Ultraschallbildgebungsvorrichtung (10) basierend auf dem Auswahlbefehl zu verriegeln,
wobei die Verriegelungseinheit (213) dafür vorgesehen ist, ein erstes Verriegeln, das den Identifikationsinformationssender (137) des Sondenverbinders (130) mit dem Identifikationsinformationsempfänger (216) der Ultraschallbildgebungsvorrichtung (10) verriegelt, und ein zweites Verriegeln, das einen Sondensignal-Sende-Empfänger (132) des Sondenverbinders (130) mit einem Körpersignal-Sende-Empfänger (212) der Ultraschallbildgebungsvorrichtung (10) verriegelt, durchzuführen und
wobei das erste Verriegeln durchgeführt wird, wenn der Sondenverbinder (130) in der Platzierungseinheit (211) platziert ist, und das zweite Verriegeln durchgeführt wird, wenn der Sondenverbinder (130) ausgewählt ist.

2. Ultraschallbildgebungsvorrichtung (10) nach Anspruch 1, wobei der Identifikationsinformationsempfänger (216) einen zweiten Kontaktpunkt aufweist, der die Sondenanordnungsidentifikationsinformationen dadurch empfängt, dass er mit dem Identifikationsinformationssender (137) einen Kontakt herstellt, der einen ersten Kontaktpunkt aufweist, der in dem Sondenverbinder (130) platziert ist.

3. Ultraschallbildgebungsvorrichtung (10) nach Anspruch 2, wobei der zweite Kontaktpunkt durch eine manuelle Bedienung eines Benutzers mit dem ersten Kontaktpunkt einen Kontakt herstellt.

4. Ultraschallbildgebungsvorrichtung (10) nach Anspruch 2, wobei die Verriegelungseinheit (213) das erste Verriegeln so durchführt, dass der erste Kontaktpunkt mit dem zweiten Kontaktpunkt einen Kontakt herstellt.

5. Ultraschallbildgebungsvorrichtung (10) nach Anspruch 1, wobei der Identifikationsinformationsempfänger (216) ein zweites drahtloses Kommunikationsmodul aufweist, das die Sondenanordnungsidentifikationsinformationen dadurch empfängt, dass es mit einem ersten drahtlosen Kommunikationsmodul des Identifikationsinformationssender (137) verbunden ist.

6. Ultraschallbildgebungsvorrichtung (10) nach Anspruch 1, welche des Weiteren folgendes aufweist:
Eine Anzeigeeinrichtung (300), welche die Sondenanordnungsidentifikationsinformationen anzeigt.

7. Ultraschallbildgebungsvorrichtung (10) nach Anspruch 1, wobei die Verriegelungseinheit (213) das erste Verriegeln zwischen einem Sondenverbinder (130), der aus der Vielzahl von Sondenverbindern (130) nicht ausgewählt ist, und der Ultraschallbildgebungsvorrichtung (10) löst.

8. Ultraschallbildgebungsvorrichtung (10) nach Anspruch 1, wobei, wenn die Eingabeeinrichtung (400) empfängt, ob sie eine erste Sondenanordnung (100-1) auswählen soll, und dann die erste Sondenanordnung (100-1) ausgewählt ist, die Verriegelungseinheit (213) ein zweites Verriegeln zwischen der ersten Sondenanordnung (100-1) und der Ultraschallbildgebungsvorrichtung (10) durchführt, und wenn die erste Sondenanordnung (100-1) nicht ausgewählt ist, die Verriegelungseinheit (213) das erste Verriegeln zwischen einer zweiten Sondenanordnung (100-2) und der Ultraschallbildgebungsvorrichtung (10) durchführt.

9. Ultraschallbildgebungsvorrichtung (10) nach Anspruch 8, wobei, wenn die erste Sondenanordnung (100-1) nicht ausgewählt ist, die Verriegelungseinheit (213) das erste Verriegeln zwischen der ersten Sondenanordnung (100-1) und der Ultraschallbildgebungsvorrichtung (10) löst.

10. Ultraschallbildgebungsvorrichtung (10) nach Anspruch 1, welche des Weiteren folgendes aufweist:
Eine Steuereinrichtung, die ein Steuersignal zum Steuern der Verriegelungseinheit (213) erzeugt, wobei die Verriegelungseinheit (213) einen Motor oder einen Aktuator aufweist, von denen beide das erste Verriegeln oder das zweite Verriegeln basierend auf dem Steuersignal ausführen.

11. Ultraschallbildgebungsvorrichtung (10) nach Anspruch 1, wobei die Platzierungseinheit (211) durch ein Kopplungsnut-Erzeugungselement realisiert ist, in welchem Kopplungsnuten so ausgebildet sind, dass eine Verriegelungswelle (133) der Sondenanordnung (110) platziert ist.

12. Ultraschallbildgebungsvorrichtung (10) nach Anspruch 11, wobei Verriegelungsnuten, in denen ein Verriegelungsstift der Verriegelungswelle (133) eingeführt sind, in dem Kopplungsnut-Erzeugungselement gebildet sind, und die Verriegelungseinheit (213) ein Kopplungselement aufweist, das an einer Position vorgesehen ist, die durch eine Verlängerung der Kopplungsnut und einen Motor gebildet ist, der die in dem Kopplungselement platzierte Verriegelungswelle (133) rotiert, wobei der Motor das Verriegeln durch Einführen des Verriegelungsstifts in die Verriegelungsnut durch Rotieren der Verriegelungswelle (133) durchführt.

13. Ultraschallbildgebungsvorrichtung (10) nach Anspruch 1, wobei die Verriegelungseinheit (213) einen Elektromagneten (218) und eine Antriebseinrichtung aufweist, wobei die Antriebseinrichtung das Verriegeln durch Bewegen des Elektromagneten (218) durchführt.

14. Steuerungsverfahren für eine Ultraschallbildgebungsvorrichtung (10), welches folgendes aufweist:
Einen Vorgang, bei dem ein Identifikationsinformationsempfänger (216) Sondenanordnungsidentifikationsinformationen von einem Identifikationsinformationssender (137) eines Sondenverbinders (130) empfängt, der in einer Platzierungseinheit (211) platziert ist;
Empfangen eines Auswahlbefehls eines Sondenverbinders (130); und
einen Vorgang, in dem eine Verriegelungseinheit (213) den Sondenverbinder (130) in eine Ultraschallbildgebungsvorrichtung (10) basierend auf dem Auswahlbefehl verriegelt,
wobei der Vorgang, in dem die Verriegelungseinheit (213) den Sondenverbinder (130) verriegelt, folgendes aufweist: Einen Vorgang, in dem die Verriegelungseinheit (213) ein erstes Verriegeln durchführt, das den Identifikationsinformationssender (137) des Sondenverbinders (130) mit dem Identifikationsinformationsempfänger (216) der Ultraschallbildgebungsvorrichtung (10) verbindet, und
ein zweites Verriegeln, das einen Sondensignal-Sende-Empfänger (132) des Sondenverbinders (130) mit einem Körpersignal-Sende-Empfänger (212) der Ultraschallbildgebungsvorrichtung (10) verbindet, und
wobei das erste Verriegeln durchgeführt wird, wenn der Sondenverbinder (130) in der Platzierungseinheit (211) platziert ist, und das zweite Verriegeln durchgeführt wird, wenn der Sondenverbinder (130) ausgewählt ist.

## Revendications

1. Appareil d'imagerie ultrasonique (10) comportant:
une unité de positionnement (211) configuré pour positionner un connecteur de sonde (130);
un récepteur d'identification d'information (216) configuré pour recevoir des informations d'identification d'ensemble de sonde à partir d'un transmetteur (137) d'identification d'informations d'un connecteur de sonde placée (130) ;
un dispositif d'entrée (400) configuré pour recevoir une commande de sélection d'un connecteur de sonde (130) ; et
une unité de fermeture (213) configurée pour fixer le connecteur de sonde (130) dans un appareil d'imagerie ultrasonique (10) sur la base de la commande de sélection,
dans lequel l'unité de fermeture (213) est configurée pour effectuer une première fermeture connectant le transmetteur (137) d'identification d'information du connecteur de la sonde (130) au récepteur d'identification d'information (216) de l'appareil d'imagerie ultrasonique (10), et une seconde fermeture connectant un récepteur (132) de signal de sonde du connecteur de sonde (130) à un transmetteur (212) de signal de corps de l'appareil d'imagerie ultrasonique (10), et
dans lequel la première fermeture est effectuée lorsque le connecteur de sonde (130) est placé dans l'unité de positionnement (211) et la seconde fermeture est effectuée lorsque le connecteur de sonde (130) est sélectionné.

2. Appareil d'imagerie ultrasonique (10) selon la revendication 1, dans lequel le récepteur d'identification d'information (216) comporte un second point de contact recevant les informations d'identification de l'assemblage de sonde en réalisant le contact avec le transmetteur (137) d'identification d'information comprenant le premier point de contact placé dans le connecteur de la sonde (130).

3. Appareil d'imagerie ultrasonique (10) selon la revendication 2, dans lequel le second point de contact effectue le contact avec le premier point de contact par une opération manuelle de l'utilisateur.

4. Appareil d'imagerie ultrasonique (10) selon la revendication 2, dans lequel l'unité de fermeture (213) accomplit la première fermeture de telle manière que le premier point de contact effectue le contact avec le second point de contact.

5. Appareil d'imagerie ultrasonique (10) selon la revendication 1, dans lequel le récepteur d'identification d'information (216) comporte un second module de communication sans fil recevant l'information d'identification de sonde en étant connecté à un premier module de communication sans fil du transmetteur (137) d'identification d'information.

6. Appareil d'imagerie ultrasonique (10) selon la revendication 1, comprenant en outre : un dispositif d'affichage (300) qui affiche les informations d'identification de l'assemblage de sonde.

7. Appareil d'imagerie ultrasonique (10) selon la revendication 1, dans lequel l'unité de fermeture (213) relâche la fermeture entre un connecteur de sonde (130) qui n'est pas sélectionné parmi la pluralité de connecteurs de sondes (130), et l'appareil d'imagerie ultrasonique (10).

8. Appareil d'imagerie ultrasonique (10) selon la revendication 1, dans lequel, lorsque le dispositif d'entrée (400) reçoit comment sélectionner un premier assemblage de sonde (100-1), et qu'ensuite le premier assemblage de sonde (100-1) est sélectionné, l'unité de fermeture (213) effectue une seconde fermeture entre le premier assemblage de sonde (100-1) et l'appareil d'imagerie ultrasonique (10), et lorsque le premier assemblage de sonde (100-1) n'est pas sélectionné, l'unité de fermeture (213) effectue la première fermeture entre un second assemblage de sonde (100-2) et l'appareil d'imagerie ultrasonique (10).

9. Appareil d'imagerie ultrasonique (10) selon la revendication 8, dans lequel, lorsque le premier assemblage de sonde (100-1) n'est pas sélectionné, l'unité de fermeture (213) relâche la première fermeture entre le premier assemblage (100-1) et l'appareil d'imagerie ultrasonique (10).

10. Appareil d'imagerie ultrasonique (10) selon la revendication 1, comprenant en outre :
un contrôleur générant un signal de contrôle pour commander l'unité de fermeture (213) dans laquelle l'unité de fermeture (213) comporte un moteur ou un actuateur, dont les deux effectuent la première fermeture ou la seconde fermeture, basées sur le signal de contrôle.

11. Appareil d'imagerie ultrasonique (10) selon la revendication 1, dans lequel l'unité de positionnement (211) est réalisée par une gorge de couplage formant un organe, dans lequel les gorges de couplage sont formées de telle manière qu'une tige de fermeture (133) de l'assemblage de sonde (110) est placée.

12. Appareil d'imagerie ultrasonique (10) selon la revendication 11, dans lequel les gorges de fermeture, dans lesquelles une goupille de fermeture de la fige de fermeture (133) est insérée, sont formées dans les membres formant les gorges de couplage, et l'unité de fermeture (213) comprend un organe de couplage disposé dans une position formée par une extension de la gorge de couplage et un moteur tournant la tige de fermeture (133) disposée dans l'organe de couplage, dans lequel le moteur effectue la fermeture en insérant la tige de fermeture dans la gorge de fermeture en tournant la tige de fermeture (133).

13. Appareil d'imagerie ultrasonique (10) selon la revendication 1, dans lequel l'unité de fermeture (213) comporte un électroaimant (218) et un dispositif d'entraînement dans lequel le dispositif d'entraînement effectue la fermeture effectue la fermeture en déplaçant l'électroaimant (218).

14. Procédé de commande d'un appareil d'imagerie ultrasonique (10), comprenant :
une opération dans laquelle un récepteur d'identification d'information (216) reçoit des informations d'identification de dispositifs de sonde d'un transmetteur (137) d'identification d'informations d'un connecteur de sonde placée (130), qui est placé dans une unité de positionnement (211) ;
la réception d'une commande sélective d'un connecteur de sonde (130) ; et
une opération dans laquelle une unité de fermeture (213) ferme le connecteur de sonde (130) dans un appareil d'imagerie ultrasonique (10) sur la base de la commande sélectionnée,
dans lequel, l'opération au cours de laquelle l'unité de fermeture (213) ferme le connecteur de sonde (130) comprend : une opération dans laquelle l'unité de fermeture (213) effectue un première fermeture connectant le transmetteur (137) d'identification d'information du connecteur de la sonde (130) au récepteur d'identification d'information (216) de l'appareil d'imagerie ultrasonique (10), et une seconde fermeture connectant un récepteur (132) de signal de sonde du connecteur de sonde (130) à un transmetteur (212) de signal de corps de l'appareil d'imagerie ultrasonique (10), et
dans lequel la première fermeture est effectuée lorsque le connecteur de sonde (130) est placé dans l'unité de positionnement (211) et la seconde fermeture est effectuée lorsque le connecteur de sonde (130) est sélectionné.
